# EUROPÄISCHE PATENTSCHRIFT

(11) **EP 0 311 963 B1**
(45) Veröffentlichungstag und Bekanntmachung des Hinweises auf die Patenterteilung: **05.05.1993**
(21) Anmeldenummer: 88116810.8
(22) Anmeldetag: 11.10.1988
(51) Int. Cl.: A61K 35/78

(54) **Therapeutisches Mittel, das einen Extrakt von Impatiens Capensis enthält**
Therapeutical medium comprising an extract of Impatiens Capensis
Agent thérapeutique comprenant un extrait d'Impatiens Capensis

(30) Priorität: 16.10.1987 DE 3735063
(43) Veröffentlichungstag der Anmeldung: 19.04.1989
(73) Patentinhaber: Lorenz, Wilfried, Dipl.-Ing., D-63069 Offenbach (DE)
(72) Erfinder: Lorenz, Wilfried, Dipl.-Ing., D-63069 Offenbach (DE)
(74) Vertreter: Schieferdecker, Lutz, Dipl.-Ing.

(56) Entgegenhaltungen:
- MEDICAL BOTANY, 1977, John Wiley & Sons, New York, NY (US); W.H.LEWIS, Seite 348*

## Beschreibung

Die Erfindung betrifft die Verwendung eines Extraktes von Impatiens Capensis als wirksame Komponente in einem Lösungsmittel oder in einem Lösungsmittelgemisch zur Herstellung eines Arzneimittels.

Es ist aus W.H. LEWIS et al.: "MEDICAL BOTANY", 1977, John Wiley & Sons, New York, USA, Seite 348, bekannt, dass ein Indianerstamm Nordamerikas den Saft von Impatiens Capensis sowohl prophylaktisch als zur Behandlung von schmerzhaften, durch giftigen Efeu hervorgerufene Wundstellen benutzt.

Der Erfindung liegt das Problem zugrunde, ein Verfahren zum Herstellen eines Arzneimittels für die Behandlung von Hautallergien, insbesondere von Neurodermatosen anzugeben.

Es wurde nun überraschenderweise gefunden, daß diese Aufgabe gemäss dem einzigen Patentanspruch mit einer Wirkstoffkombination gelöst wird, die in der Pflanze Impatiens Capensis enthalten ist und zum Beispiel mit einem alkoholischen Lösungsmittel oder Lösungsmittelgemisch extrahiert werden kann.

Impatiens Capensis ist eine Vertreterin der Springkrautgewächse und in weiten Teilen Nordamerikas verbreitet.

Die Behandlung von Hautallergien mit Hilfe eines Arzneimittels, das unter Verwendung eines Extraktes von Impatiens Capensis hergestellt wird, gestattet es, in vielen Fällen auf die Verwendung von kortisonhaltigen Präparaten zu verzichten. Die von Kortisonbehandlungen her bekannten Nebenwirkungen treten bei der Wirkstoffkombination der Pflanze Impatiens Capensis nicht auf, so dass es sich hier um eine wertvolle Alternative zu Kortison handelt.

Bei der Behandlung der Haut wird die Wirkstoffkombination lokal mit vorteilhafter Wirkung als Salbe, Creme, Gelee oder Tinktur angewendet. Die Formulierung der jeweiligen Verarbeitungsform erfolgt nach üblichen Methoden unter Verwendung üblicher Mittel und Hilfsstoffe.

Die Erfindung wird nachstehend anhand eines Beispieles nochmals näher erläutert.

### Beispiel

### Extraktionsverfahren

Die Extraktgewinnung erfolgt im Rahmen eines Frischauszuges aus der Pflanze, d.h. ihren Blättern, Stielen und Blüten in Verbindung mit Alkohol. Die Pflanze wird zunächst zerrieben. Sodann wird z.B. 70prozentiger Alkohol hinzugegeben. Dieser verdunstet, woraufhin der Frischauszug mit einer Basiscreme gemischt wird.

Im konkreten Fall werden 100 Gramm Pflanzensubstanz mit dem Messer kleingeschnitten und in ein Gefäss gegeben. Sodann werden 500 Milliliter eines 70prozentigen Alkohols hinzugegeben. Etwa die Hälfte dieser Alkoholmenge verdunstet innerhalb von zwei bis drei Stunden. Um die Substanz während der nächsten 14 Tage bis drei Wochen flüssig zu halten, werden 250 Milliliter Wasser hinzugegeben. Nach 14 Tagen bis drei Wochen wird die dann noch vorhandene Flüssigkeit abgegossen bzw-. abgesiebt, in der praktisch kein Alkohol mehr vorhanden ist. Der in Wasser gelöste Extrakt wird sodann mit 100 Milliliter wasserlöslicher Basiscreme als Träger gemischt und kann daraufhin zur Behandlung verwendet werden.

Die Zugabe der 250 Milliliter Wasser erfolgt , damit der Extrakt nicht nur als Kruste am Gefäss antrocknet und sich nur mit Stahlwolle abkratzen lässt , sondern damit der Extrakt in einer leicht weiter verarbeitbaren Form anfällt.

Mit dem derart gewonnenen Extrakt durchgeführte Behandlungen von akuten Neurodermatosen waren überraschenderweise nach kurzer Zeit erfolgreich, ohne dass irgendwelche Nebenwirkungen zu beobachten gewesen wären.

## Patentansprüche

1. Verwendung eines Extraktes von Impatiens Capensis als wirksame Komponente in einem Lösungsmittel oder in einem Lösungsmittelgemisch zur Herstellung eines Arzneimittels für die Behandlung von Hautallergien, insbesondere von Neurodermatosen.

## Claims

1. Use of an extract from Impatiens Capensis as an active component in a solvent or in a solvent mixture for the preparation of a medicament for the treatment of skin allergies, in particular neurodermatoses.

## Revendications

1. Utilisation d'un extrait d'Impatiens Capensis, en tant que composant efficace dans une solution ou dans un mélange de solution pour la fabrication d'un médicament pour le traitement d'allergies de la peau, en particulier de dermatoses névrotiques.
